(19) Europäisches Patentamt
European Patent Office
Office européen des brevets

(11) EP 3 533 779 B1

(12) **EUROPEAN PATENT SPECIFICATION**

(45) Date of publication and mention of the grant of the patent:
**05.04.2023 Bulletin 2023/14**

(21) Application number: **17865839.9**

(22) Date of filing: **13.07.2017**

(51) International Patent Classification (IPC):
***C07C 67/333*** *(2006.01)* ***C07C 69/75*** *(2006.01)*
***B01J 23/02*** *(2006.01)* ***C07B 61/00*** *(2006.01)*
***C07C 69/757*** *(2006.01)*

(52) Cooperative Patent Classification (CPC):
(C-Sets available)
**C07C 69/757; C07C 67/333;** B01J 23/02;
C07B 61/00; C07C 2601/14 (Cont.)

(86) International application number:
**PCT/JP2017/025540**

(87) International publication number:
**WO 2018/078961 (03.05.2018 Gazette 2018/18)**

(54) **PRODUCTION METHOD FOR TRANS-BIS(2-HYDROXYALKYL) CYCLOHEXANEDICARBOXYLATE, AND BIS(2-HYDROXYALKYL) CYCLOHEXANEDICARBOXYLATE**

VERFAHREN ZUR HERSTELLUNG VON TRANS-BIS(2-HYDROXYALKYL)CYCLOHEXANDICARBOXYLAT UND BIS(2-HYDROXYALKYL)CYCLOHEXANDICARBOXYLAT

PROCÉDÉ DE PRODUCTION DE TRANS-BIS(2-HYDROXYALKYL) CYCLOHÉXANEDICARBOXYLATE, ET BIS(2-HYDROXYALKYL) CYCLOHÉXANEDICARBOXYLATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2016 JP 2016211797**
**13.06.2017 JP 2017115590**

(43) Date of publication of application:
**04.09.2019 Bulletin 2019/36**

(73) Proprietor: **Jeplan, Inc.**
**Kawasaki-shi, Kanagawa 210-0867 (JP)**

(72) Inventors:
- **ITO, Hiroshi**
**Kizugawa-shi**
**Kyoto 619-0225 (JP)**
- **IMANAKA, Hiroshi**
**Funabashi-shi**
**Chiba 273-0853 (JP)**
- **INADA, Shuji**
**Suita-shi**
**Osaka 565-0873 (JP)**

(74) Representative: **Hoffmann Eitle**
**Patent- und Rechtsanwälte PartmbB**
**Arabellastraße 30**
**81925 München (DE)**

(56) References cited:
**JP-A- 2000 191 602** **JP-A- 2000 191 602**
**JP-A- 2009 126 854** **JP-A- 2010 270 093**
**US-A- 5 817 731** **US-A- 5 817 731**

- **DANFENG HOU ET AL: "Conversion of bis(2-hydroxyethylene terephthalate) into 1,4-cyclohexanedimethanol by selective hydrogenation using RuPtSn/Al 2O3", RSC ADVANCES, vol. 6, no. 54, 1 January 2016 (2016-01-01), pages 48737-48744, XP055480031, DOI: 10.1039/C6RA04943E**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 533 779 B1

• **HOU, DANFENG ET AL.: 'Conversion of bis(2-hydroxyethylene terephthalate) into 1,4-cyclohexanedimethanol by selective hydrogenation using RuPtSn/A1203' RSC ADVANCES vol. 6, no. 54, 12 May 2016, pages 48737 - 48744, XP055480031**

(52) Cooperative Patent Classification (CPC): (Cont.)

C-Sets
**C07C 67/333, C07C 69/757**

## Description

## BACKGROUND OF THE INVENTION

### Technical Field

The present invention relates to a process for producing trans-cyclohexanedicarboxylic acid bis(2-hydroxyalkyl) ester and to cyclohexanedicarboxylic acid bis (2-hydroxyalkyl) ester.

### Background Art

Consumption of polyethylene terephthalate (hereinafter referred to as "PET") products is increasing year by year, and improving the recycling rate of waste PET products is an essential proposition on a global scale. As a recycling method of waste PET products, it is known, for example, a method of obtaining terephthalic acid bis (2-hydroxyethyl) ester (hereinafter referred to as "BHET") from a waste PET product and recycling it as a raw material of PET.

BHET is also used as a raw material for 1,4-cyclohexanedicarboxylic acid bis (2-hydroxyethyl) ester (hereinafter referred to as "BHEC") besides recycling as a raw material for PET. BHEC can be produced, for example, by reacting BHET with hydrogen (hydrogen gas) to hydrogenate (nuclear hydrogenate) the benzene ring of BHET. As a method for obtaining BHEC from BHET, there is, for example, a method disclosed in Patent Document 1 as referred to below. According to the method disclosed in Patent Document 1, BHEC is produced by reacting BHET with hydrogen gas in the presence of a palladium-supported catalyst to perform nuclear hydrogenation of BHET.

Incidentally, BHEC includes two stereoisomers, such as, cis-1,4-cyclohexanedicarboxylic acid bis (2-hydroxyethyl) ester (hereinafter referred to as "cis-BHEC") and trans-1,4-cyclohexanedicarboxylic acid bis (2-hydroxyethyl) ester (hereinafter referred to as "trans-BHEC"). However, according to the analysis by the present inventors, only cis-BHEC was detected from BHEC produced by the method of Patent Document 1, and trans-BHEC was not detected. There is also not known a method for isomerizing cis-BHEC to obtain trans-BHEC.

Cis-BHEC is known as a raw material for polyester resin, polyurethane resin, acrylic resin, epoxy resin and the like. On the other hand, since trans-BHEC has high linearity, it is expected to be an excellent raw material as, for example, a chain extender (diol component) of polyurethane resin. It is therefore desired a development of a method of obtaining trans-BHEC.

## PRIOR ART DOCUMENTS

### Patent Documents

Patent Document 1: CN104003840 (A). Document US5817731A discloses trans bis(2-hydroxyalkyl)-1,4-cyclohexanedicarboxylate.

## SUMMARY OF THE INVENTION

### Problems to be solved by The Invention

The present invention has been made in consideration of the conventional problems as stated above, and is aimed at a process for simply producing trans-cyclohexanedicarboxylic acid bis (2-hydroxyalkyl) ester in high yield.

### Means to solve The Problems

The present invention includes:

(1) A process for producing trans-cyclohexanedicarboxylic acid bis (2-hydroxyalkyl) ester comprising a step of isomerizing cis-cyclohexanedicarboxylic acid bis (2-hydroxyalkyl) ester by contacting with a basic oxide to obtain trans-cyclohexanedicarboxylic acid bis (2-hydroxyalkyl) ester.

(2) The process according to the above (1), wherein the isomerization is carried out at temperatures of 160 to 300 °C.

(3) The process according to the above (1) or (2), wherein the weight ratio of the cis-cyclohexanedicarboxylic acid bis (2-hydroxyalkyl) ester to the basic oxide is 100 : 0.1 to 100 : 10.

(4) The process according to any one of (1) to (3) above, wherein the isomerization is carried out in the state that the cis-cyclohexanedicarboxylic acid bis (2-hydroxyalkyl) ester and the basic oxide are mixed in a liquid medium.

(5) The process according to (4) above, wherein the liquid medium comprises at least one of a diol and an aprotic

polar solvent.

(6) The process according to (5) above, wherein the liquid medium comprises at least one of ethylene glycol, propylene glycol, butanediol, N, N-dimethylformamide, dimethylsulfoxide and 1,3-dimethyl-2-imidazolidinone.

(7) The process according to any one of (1) to (6) above, wherein the basic oxide comprises at least one of alkali metal oxides and alkaline earth metal oxides.

(8) The process according to any one of (1) to (7) above, wherein the basic oxide comprises at least one of barium oxide, calcium oxide and magnesium oxide.

(9) The process according to any one of the above (1) to (8), wherein the cis-cyclohexanedicarboxylic acid bis (2-hydroxyalkyl) ester is cis-1,4-cyclohexanedicarboxylic acid bis (2-hydroxyethyl) ester.

(10) The process according to (9) above, wherein the cis-1 ,4-cyclohexanedicarboxylic acid bis (2-hydroxyethyl) ester is produced by depolymerizing a raw material containing polyethylene terephthalate to obtain terephthalic acid bis (2-hydroxyethyl) ester and then nuclear hydrogenating the terephthalic acid bis (2-hydroxyethyl) ester.

(11) The process according to (10) above, wherein the nuclear hydrogenation is carried out by reacting the terephthalic acid bis (2-hydroxyethyl) ester with hydrogen gas in the presence of a ruthenium catalyst.

(12) The process according to the above (11), wherein the nuclear hydrogenation is carried out in a state where the terephthalic acid bis (2-hydroxyethyl) ester and the ruthenium catalyst are mixed in a liquid medium.

## Effect of The Invention

According to the present invention, the trans-cyclohexanedicarboxylic acid bis (2-hydroxyalkyl) ester can be simply obtained in high yield from the cis-cyclohexanedicarboxylic acid bis (2-hydroxyalkyl) ester. It is also possible to provide the cyclohexanedicarboxylic acid bis (2-hydroxyalkyl) ester containing a high proportion of the trans-cyclohexanedicarboxylic acid bis (2-hydroxyalkyl) ester.

## BRIEF DESCRIPTION OF THE DRAWINGS

FIG.1 is a gas chromatogram of a reaction mixture obtained in Example 1.
FIG. 2 is a gas chromatogram of a reaction mixture obtained in Example 3.
FIG.3 is a gas chromatogram of a reaction mixture obtained in Example 4.

## BEST MODE FOR CARRYING OUT THE INVENTIO

Hereinafter, preferred embodiments of the present invention, i.e., the process for producing trans-cyclohexanedicarboxylic acid bis (2-hydroxyalkyl) ester will be described in detail.

The process for producing the trans-cyclohexanedicarboxylic acid bis (2-hydroxyalkyl) ester (hereinafter referred to as "trans-BHAC") according to the present invention is a process for producing the trans-BHAC by isomerizing cis-cyclohexanedicarboxylic acid bis (2-hydroxyalkyl) ester (hereinafter referred to as "cis-BHAC ") by contact with a basic oxide.

More specifically, the process for producing trans-BHAC according to the present invention includes steps of preparing a raw material containing cis-BHAC (raw material preparation step), and isomerizing the cis-BHAC by charging the raw material together with the basic oxide into the autoclave to obtain the trans-BHAC by contacting the cis-BHAC with the basic oxide (isomerization step). Each step will be described in turn below.

<Raw Material Preparation Step>

First, a raw material containing cis-BHAC is prepared.

A specific example of the cis-BHAC includes a chemical compound represented by the following general formula (1).

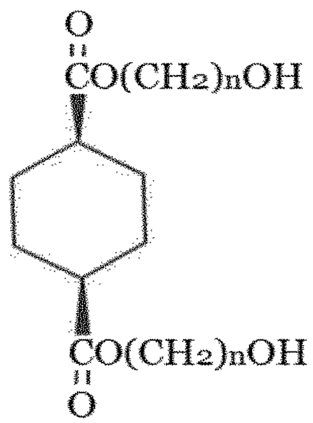

(1)

(In the general formula (1), "n" represents an integer of 2 or more.)

In the above general formula (1), an ester group is bonded to the 1-position and the 4-position of the cyclohexane ring, but the present invention is not limited thereto. Examples of the cis-BHAC used in the present invention include cis-1,4-cyclohexanedicarboxylic acid bis (2-hydroxyalkyl) ester represented by the above general formula (1), cis-1,3-cyclohexanedicarboxylic acid bis (2-hydroxyalkyl) ester, cis-1,2-cyclohexanedicarboxylic acid bis (2-hydroxyalkyl) ester and the like. Among them, cis-1,4-cyclohexanedicarboxylic acid bis (2-hydroxyalkyl) ester has high linearity, so it is an excellent raw material as a chain extender (diol component) of polyurethane resin, for example.

In the above general formula (1), the value of "n" is not particularly limited as long as it is an integer of 2 or more. However, the value of "n" is preferably 2 to 12, more preferably 2 to 6. A cis-BHAC having a value of "n" within the above range is easy to handle and excellent in productivity. By using such cis-BHAC as a raw material, side reactions other than the isomerization reaction can be more reliably suppressed, so that highly-purified trans-BHAC can be obtained with higher yield.

As raw materials containing such cis-BHAC, commercially available products and synthetic products can be used. In particular, the raw material containing cis-1,4-cyclohexanedicarboxylic acid bis (2-hydroxyethyl) ester (BHEC) is obtained by depolymerizing waste PET products to obtain terephthalic acid bis (2-hydroxyethyl) ester (BHET) and then nuclear hydrogenating the BHET. By using such a raw material, the recycling rate of the waste PET products can be improved. Examples of the waste PET products include waste PET bottles, waste PET films, waste PET fibers, waste PET industrial materials, and the like.

Hereinafter, a method for obtaining a raw material containing cis-BHEC from the waste PET products will be described specifically.

A method of obtaining a raw material containing cis-BHEC from waste PET products (cis-BHEC production method) comprises a step of obtaining a reactant containing BHET by depolymerizing the waste PET product (depolymerization step) and a step of nuclear hydrogenating the BHET by charging the obtained reactant together with a catalyst into the autoclave to obtain cis-BHEC by reacting BHET contained in the reactant and hydrogen gas in the presence of the catalyst (nuclear hydrogenation step).

<< Depolymerization Step >>

First, as a method of obtaining BHET from the waste PET product, a method of depolymerizing PET in ethylene glycol (hereinafter referred to as "EG") is known from, for example, JP2000-53802A and JP2008-88096A. According to such a method, it is possible to obtain high purity BHET from the inexpensive waste PET product with high yield. In addition, such a method is also a technology greatly contributing to the protection of the global environment since it is chemical recycling that makes effective use of resources.

The content of BHET in the reaction product (purity of BHET) obtained by depolymerization of PET is preferably 90 wt% or more, 92 wt% or more is more preferable and 95 wt% or more is still more preferable. By using the reactant whose purity of BHET is within the above range, cis-BHEC of higher purity can be obtained in good yield.

<< Nuclear Hydrogenation Step >>

Next, the PET depolymerization reaction product and the catalyst are charged (supplied) into a vessel (reactor). Thereafter, a reaction gas containing hydrogen gas is filled in the vessel to react BHET and hydrogen gas in the presence of the catalyst. As a result, the benzene ring of BHET is hydrogenated (also called "nuclear hydrogenated") to obtain a raw material containing cis-BHEC.

As the reactor, for example, an autoclave equipped with a stirrer can be used.

The catalyst acts to efficiently hydrogenate the benzene ring of BHET. Such a catalyst is not particularly limited, but it is preferable to include at least one of a noble metal and a noble metal alloy.

Specific examples of such catalysts include noble metals such as Ru, Rh, Pd, Os, Ir and Pt, alloys of two or more noble metals, alloys of noble metals with at least one of metal elements such as Ni, Co, Fe, Zn, Cu, Mn, Pd, Cd, Cr, Ag, Au, Hg, Ga, In, Ge, Sn, Ti, Al and Si, alkaline earth metal elements of Ca, Mg, Gr and Ba, and alkali metals of Li, Na, K, Rb and Cs. Among these metals, it is particularly preferable to contain Ru (ruthenium) for the catalyst.

The ruthenium catalyst can selectively hydrogenate the benzene ring of BHET. In addition, since the ruthenium catalyst can suppress the hydrogenation of the ester group ($-COOCH_2CH_2$ OH) of BHET, it is possible to suppress the generation of 1,4-cyclohexanedimethanol (hereinafter referred to as "CHDM") in which both of a benzene ring and an ester group are hydrogenated from BHET. Therefore, by using the ruthenium catalyst, generation of by-products (such as, CHDM) can be suppressed and cis-BHEC with high purity can be obtained in good yield. Furthermore, unexpectedly, it was found that BHEC produced by nuclear hydrogenation of BHET in the presence of ruthenium catalyst contains trans-BHEC in addition to cis-BHEC. That is, it was revealed that in the presence of the ruthenium catalyst, it is possible to obtain high-purity BHEC in good yield by the reaction between BHET and hydrogen gas and that the obtained BHEC contains cis-BHEC and trans-BHEC.

In this step, a noble metal or a noble metal alloy (hereinafter simply referred to as "noble metal") can be used as it is, but it is preferable to use it as a noble metal supported carrier catalyst carrying a noble metal on a carrier. The carrier adsorbs the noble metal and disperses the point acting as a catalyst (catalyst active points) on the surface of the carrier. As a result, the contact area between the catalyst active point and BHET can be increased, so that the efficiency of nuclear hydrogenation of BHET can be further enhanced. Moreover, by supporting a noble metal on a carrier, durability of the catalyst can be enhanced.

Also, as a carrier for supporting a noble metal, a carrier containing at least one of carbon (activated carbon), alumina, silica, titania, magnesia, zirconia, silica alumina, zeolite, clay, kaolin, talc and bentonite is preferable. Among them, a carrier containing at least one of carbon, alumina, silica, titania, magnesia and zirconia is more preferable, and a carrier containing carbon is particularly preferable.

The amount (content) of the noble metal supported on the carrier is not particularly limited, but it is preferably about 0.1 to 10 wt%, and more preferably about 0.5 to 10 wt%. When the amount of the noble metal supported on the noble metal-supported carrier is within the above range, it is possible to obtain a noble metal-supported carrier catalyst in which the catalyst active points are uniformly dispersed on the surface of the carrier while suppressing the use amount of expensive noble metal. By using such a noble metal-supported carrier catalyst, BHET can be nuclear hydrogenated efficiently at low cost, and high purity BHEC can be obtained in good yield. In addition, since such a ruthenium-supported carrier catalyst has high durability as described above, it can be recycled as a catalyst by recovering it separately.

From the viewpoint of improving the catalytic action of the noble metal, an activation treatment of heating the noble metal may be performed in advance in various gas flows such as hydrogen, nitrogen, argon, carbon dioxide or the like. The treatment temperature at that time is preferably about 50 to 700 °C., and more preferably about 80 to 600 °C. When using a noble metal-supported carrier catalyst, for example, by heating at a temperature within the above temperature range, the catalyst active points of the noble metal is uniformly dispersed over the surface of the carrier, so that the catalytic characteristics of the noble metal-supported carrier catalyst can be further enhanced. The time of the activation treatment can be appropriately adjusted depending on the amount of noble metal supported on the carrier and the treatment temperature, but it can be set to, for example, about 0.1 to 100 hours.

The charging ratio between BHET and catalyst charged into the autoclave (reactor) is not particularly limited, but it is preferably 100: 0.1 to 100: 10 in terms of weight ratio of BHET and catalyst, and more preferably 100: 1 to 100: 5. This makes it possible to nuclear hydrogenate BHET efficiently in a shorter time and to more reliably suppress side reactions (such as hydrogenation reaction of ester group of BHET) other than nuclear hydrogenation reaction of BHET. As a result, BHEC with higher purity can be obtained in good yield.

Incidentally, in the nuclear hydrogenation reaction of BHET, it is desirable to increase contact between BHET and hydrogen and/or catalyst as much as possible. However, since the melting point of BHET is relatively high (about 110 °C.), a large amount of heat is required to increase the fluidity of BHET by heating. Therefore, in order to improve the fluidity of BHET, it is effective to dissolve or disperse BHET in a liquid medium. By using the liquid medium, heat of reaction can be reduced, so that side reactions can be suppressed more reliably. When using a liquid medium in this step, it is preferable to charge the PET depolymerization reaction product, the catalyst and the liquid medium into the autoclave and stir them by an agitator of the autoclave so that the nuclear hydrogenation of BHET can be carried out in the state that the PET depolymerization reaction product and the catalyst are dissolved or dispersed in the liquid medium.

The liquid medium usable in this nuclear hydrogenation step is not particularly limited as long as it does not adversely affect the nuclear hydrogenation reaction of BHET, but it is preferable that it contains at least one kind of diol and aprotic polar solvent. Since these liquid media have high solubility in BHET, the fluidity of BHET can be improved. In addition, it is possible to suppress the occurrence of side reactions such as polymerization during the nuclear hydrogenation reaction of BHET. Therefore, by using such a liquid medium, BHEC with higher purity can be obtained in good yield.

The diol is not particularly limited, but it is preferable to include at least one kind of ethylene glycol, propylene glycol, butane diol and the like. Also, the aprotic polar solvent is not particularly limited, but it is preferable to include at least one of N, N-dimethylformamide, dimethyl sulfoxide and 1,3-dimethyl-2-imidazolidinone. Among these liquid media, ethylene glycol is particularly preferable from the viewpoint that it is a diol constituting BHET, the solubility of BHET being high and the effect of suppressing side reaction such as polymerization being high.

Although the amount (amount of use) of the liquid medium to be charged into the autoclave is not particularly limited, it is preferably 5:95 to 90:10, and more preferably 10:90 to 70:30 in terms of weight ratio of BHET and the liquid medium. When the amount of the liquid medium used falls within the above range, the nuclear hydrogenation reaction of BHET proceeds efficiently and side reactions can be effectively suppressed. As a result, the productivity of BHEC can be improved with higher yield of BHEC.

After introducing the raw material and the catalyst into the autoclave equipped with the stirrer, the interior of the autoclave is evacuated (vacuum degassed) and then filled with a reaction gas containing hydrogen gas, or an air in the autoclave is replaced with reaction gas containing hydrogen gas to thereby react BHET with hydrogen gas in the presence of ruthenium.

Note that these gas substitution operation and vacuum degassing operation may be performed in combination, or these operations may be repeated alternately and repeatedly. The reaction gas to be filled in the autoclave may be a single hydrogen gas, or a mixed gas of nitrogen, argon, carbon dioxide gas and hydrogen gas.

In the case of using a mixed gas, the partial pressure of hydrogen gas (the total pressure when using hydrogen gas alone) is not particularly limited, but preferably about 1 to 10 MPa, more preferably about 2 to 8 MPa, and furthermore preferably about 3 to 6 MPa. When the partial pressure of hydrogen gas is within the above range, hydrogen gas more selectively reacts with the benzene ring of BHET, and nuclear hydrogenation of BHET proceeds more rapidly. As a result, BHEC can be obtained efficiently. Furthermore, with such a relatively low partial pressure, since the burden on the autoclave is suppressed, it is possible to manufacture BHEC repeatedly in the same facility for a long period of time, so that the running cost can be suppressed. Note that during nuclear hydrogenation of BHET, it is preferable to supply hydrogen gas continuously or intermittently into the autoclave so that the partial pressure of hydrogen gas in the autoclave can become constant.

The reaction temperature (temperature in the autoclave) at the time when BHET is reacted with hydrogen gas is preferably about 80 to 200 °C., more preferably about 85 to 180 °C., and furthermore preferably about 80 to 170 °C. If the reaction temperature is within the above range, hydrogen gas more selectively reacts with the benzene ring of BHET and the nuclear hydrogenation of BHET proceeds promptly. In addition, when the reaction temperature is within the above range, it is possible to more reliably suppress side reactions, such as hydrogenation reaction of ester group of BHET, etc., other than the nuclear hydrogenation reaction of BHET. As a result, high purity BHEC can be obtained in higher yield. Also, with such relatively low reaction temperature, since the burden on the autoclave is reduced, the running cost can be suppressed.

The reaction time of BHET and hydrogen gas is appropriately adjusted depending on the type of catalyst to be used, the charging ratio of the raw material and the catalyst, the pressure of the reaction gas (partial pressure of hydrogen gas), the reaction temperature, etc., and is thus not particularly limited, but may be about 2 to 20 hours, for example.

Further, the reaction mode for obtaining BHEC can be carried out in a batch type, a continuous type, or a combination of the batch and the continuous types. As a batch type hydrogenation apparatus, a reactor (autoclave) equipped with a stirrer and a cooler such as a total reflux condenser can be used. Also, a known continuous fixed bed reactor can be used as a continuous hydrogenation apparatus.

After carrying out the hydrogenation reaction as described above, it is cooled down to room temperature (about 20 °C) if necessary. Thereafter, by separating the catalyst from the reaction system in the autoclave, crude BHEC can be obtained. The content of BHEC in such a crude BHEC is preferably 80 wt% or more. According to the above method, generation of by-products is suppressed, and BHEC with high purity can be obtained.

The crude BHEC includes cis-BHEC and trans-BHEC. The weight ratio of cis-BHEC to trans-BHEC in the crude BHEC is preferably 99: 1 to 35:65. By using a raw material including cis-BHEC and trans-BHEC at such a weight ratio, it is possible to further increase the yield of trans-BHEC ultimately obtained after an isomerization step.

The crude BHEC can be purified by a known separation and purification method such as distillation or recrystallization.

Through the above steps, a raw material containing cis-BHEC can be obtained. As in cis-BHEC, cis-BHAC can be also obtained by nuclear hydrogenation of terephthalic acid bis (2-hydroxyalkyl) ester (BHAT).

<Isomerization Step>

Next, the raw material containing cis-BHAC and basic oxide are brought into contact with each other by feeding

(supplying) the raw material containing cis-BHAC into a vessel (reactor). As a result, trans-BHAC is obtained by isomerization of cis-BHAC. See the following general formula (2). In the general formula (2), an ester group is bonded to the 1-position and the 4-position of the cyclohexane ring, but like the formula (1), the present invention is not limited thereto.

[Chemical formula 2]

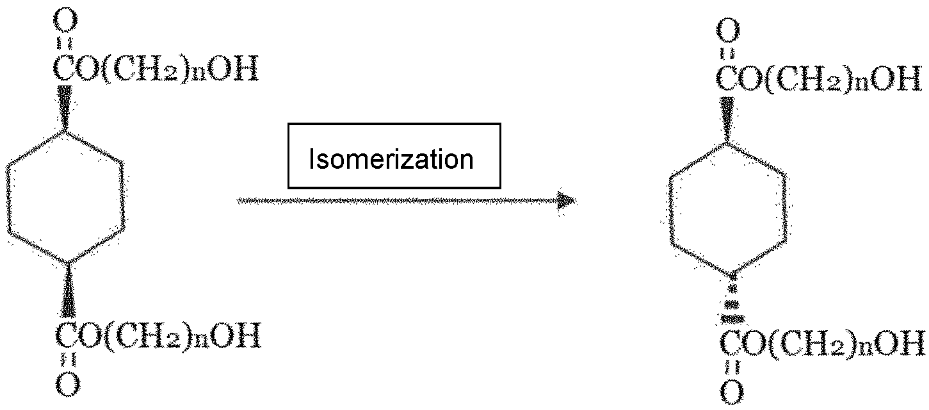


(2)

(In the general formula (2), "n" represents an integer of 2 or more.)

As the reactor, an autoclave equipped with a stirrer, for example, can be used.

In the process for producing trans-BHAC according to the present invention, the key feature is that a basic oxide is used as a catalyst (isomerization catalyst) for isomerizing cis-BHAC. That is, cis-BHAC can be rapidly isomerized to trans-BHAC by contacting the basic oxide with cis-BHAC. In addition, by using a basic oxide, it is possible to suppress side reactions (such as, hydrolysis reaction of ester, dehydration condensation reaction of hydroxyl group, etc.) other than the isomerization reaction of cis-BHAC. As a result, trans-BHAC with high purity can be obtained in good yield. Further, the basic oxide does not corrode the manufacturing apparatus (reactor) unlike the acidic catalyst. Therefore, in the method for producing trans-BHAC of the present invention, since the burden on the reactor is minimized, it is possible to manufacture trans-BHAC repeatedly in the same facility for a long period of time, and it is possible to reduce the running cost.

As stated above, the basic oxide acts as a catalyst for efficiently isomerizing cis-BHAC.

Specifically, the basic oxide is an oxide of a metal element. Such a metal element is not particularly limited, but it preferably comprises at least one kind of alkali metal and alkaline earth metal. By using an oxide of an alkaline earth metal or an oxide of an alkali metal as a catalyst, it is possible to efficiently isomerize cis-BHAC, and reliably suppress side reactions, such as, ester hydrolysis reaction, etc. In particular, from the viewpoint of accelerating a rapid isomerization reaction of cis-BHAC, it is preferable to use an oxide of an alkaline earth metal as the basic oxide.

The oxide of the alkaline earth metal is more preferable, if it contains at least one of barium oxide, calcium oxide and magnesium oxide.

Incidentally, as a method of bringing the basic oxide into contact with cis-BHAC, a suspension bed method and a fixed bed method, for example, can be mentioned. As for the suspension bed system, it is preferable to use a basic oxide powder, and in the case of the fixed bed system, it is preferable to use a molded article by molding a basic oxide.

As the basic oxide powder, a powder having an arbitrary particle size distribution can be used. The shape of the molded article is not particularly limited, but from the viewpoint of easiness of molding, it is preferable that the shape of the molded article is cylindrical.

The charging ratio of cis-BHAC and basic oxide charged into the autoclave (reactor) is not particularly limited, but it is preferable that the weight ratio of cis-BHAC and basic oxide is 100: 0.1 to 100:10, and more preferably 100:0.5 to 100: 5. This makes it possible to isomerize cis-BHAC efficiently in a shorter time and more reliably suppress side reactions other than the isomerization reaction of cis-BHAC, such as, ester hydrolysis reaction, etc. As a result, trans-BHAC with higher purity can be obtained in good yield.

In the isomerization reaction of cis-BHAC, it is desirable to increase contact between cis-BHAC and basic oxide as much as possible. However, since the melting point of cis-BHAC is relatively high, a large amount of heat is required to increase the fluidity of cis-BHAC by heating. Therefore, in order to improve the fluidity of cis-BHAC, it is effective to dissolve or disperse cis-BHAC in a liquid medium. In addition, by using a liquid medium, heat of reaction can be reduced, and side reactions can be suppressed more reliably. When using a liquid medium in this step, the raw materials, the basic oxide and the liquid medium are charged into the autoclave and stirred by an agitator of an autoclave. As such, the isomerization of cis-BHAC should preferably be performed in the state that the raw material and cis-BHAC are

dissolved and dispersed in the liquid medium.

The liquid medium usable in this step is not particularly limited as long as it does not adversely affect the isomerization reaction of cis-BHAC, but diol such as ethylene glycol, propylene glycol and butanediol, N, N-dimethylformamide, dimethyl sulfoxide and 1,3-dimethyl-2-imidazolidinone is preferable as a liquid medium containing at least one of aprotic polar solvents such as sulfoxide and 1,3-dimethyl-2-imidazolidinone. Among these liquid mediums, ethylene glycol is particularly preferable from the viewpoint of high compatibility with cis-BHAC and high effect of suppressing side reactions.

The amount of the liquid medium to be charged into the autoclave is not particularly limited, but it is preferably 20:80 to 99: 1 and more preferably 20:80 to 90:10 in terms of the weight ratio of cis-BHAC to the liquid medium. When the amount of the liquid medium used falls within the above range, the isomerization reaction of cis-BHAC proceeds efficiently and side reactions can be effectively suppressed. As a result, the productivity of trans-BHAC improves and the yield of trans-BHAC can be increased.

The isomerization reaction of cis-BHAC is preferably carried out in an atmosphere of an inert gas. By doing so, even when isomerization of cis-BHAC is carried out at high temperature, it is possible to more reliably prevent oxidation and deterioration of raw materials, produced trans-BHAC and liquid medium. As the inert gas, a gas containing at least one of nitrogen gas and argon gas is preferable. Specifically, after introducing the raw material and the basic oxide into an autoclave equipped with a stirrer, the interior of the autoclave is evacuated (vacuum degassed) and then filled with the inert gas, or the air in the autoclave is substituted by the inert gas. As a result, cis-BHAC can be isomerized under an inert gas atmosphere.

This step can be performed in a state where the pressure inside the autoclave is normal pressure (atmospheric pressure), but in the case where the liquid medium to be used has a low boiling point, pressure may be added. When pressurizing the inside of the autoclave, the preferable pressure in the autoclave is, for example, about 1 to 10 MPa.

The reaction temperature (temperature in the autoclave) for isomerizing cis-BHAC is not particularly limited, but it is preferably about 160 to 300 °C, more preferably about 180 to 280 °C, and even more preferably 180 to 240 °C. When the reaction temperature is within the above range, isomerization of cis-BHAC proceeds promptly. In addition, if the reaction temperature is within the above range, the lower temperature more reliably suppresses side reactions, such as, ester hydrolysis reaction, dehydration condensation reaction, etc. other than the isomerization reaction of cis-BHAC, and it is also possible to suppress the coloration of the reaction mixture. As a result, high purity trans-BHAC can be obtained with higher yield. Also, at such relatively low reaction temperature, since the burden on the autoclave is reduced, the running cost can be reduced too. Particularly, when the reaction temperature is about 160 to 200 °C, a low boiling point solvent can be used as the liquid medium, and furthermore, it can react at normal pressure (atmospheric pressure). Therefore, it is unnecessary to use a high pressure apparatus, and a relatively inexpensive reaction apparatus can be used, which is industrially advantageous.

The reaction time for isomerizing cis-BHAC is appropriately adjusted depending on the type of the basic oxide to be used, the charging ratio of the raw material and the basic oxide, the pressure in the autoclave, the reaction temperature, etc., and is not particularly limited. However, it can be set to about 2 to 20 hours, for example.

In addition, like the above-described nuclear hydrogenation reaction of BHET, the reaction mode for obtaining trans-BHAC can be carried out by a batch type, a continuous type, or a combination of the batch and the continuous types.

After the isomerization reaction is carried out as described above, it is cooled to room temperature (about 20 °C) as required. Thereafter, the basic oxide is separated from the reaction product in the autoclave, whereby the crude trans-BHAC can be obtained. The content of trans-BHAC in such crude transformer-BHAC is preferably 60 wt% or more. That is, it is possible to obtain BHAC according to this embodiment having a content of trans-BHAC of 60 wt% or more. As such, according to the method of producing the transformer-BAC of this embodiment, generation of by-products is suppressed, and trans-BHAC of high purity can be obtained.

In addition, the obtained crude trans-BHAC can be purified using known separation and purification methods such as distillation and recrystallization.

Incidentally, the basic oxide separated from the reaction product can be reused repeatedly. That is, it is possible to obtain trans-BHAC by introducing a new raw material and a basic oxide recovered separately from the reaction product into the autoclave and isomerizing cis-BHAC in the same manner as described above. Thus, even when the used basic oxide is recycled, it is possible to efficiently and repeatedly isomerize cis-BHAC to obtain highly pure trans-BHAC with high yield.

Although the process for producing trans-cyclohexanedicarboxylic acid bis (2-hydroxyalkyl) ester according to the present invention has been described above, the present invention is not limited thereto, and one or more steps for arbitrary purpose may be added.

## EXAMPLES

Hereinafter, specific examples of the present invention will be described.

(Example 1)

First, a raw material containing cis-1,4-cyclohexanedicarboxylic acid bis (2-hydroxyethyl) ester (cis-BHEC) was obtained from a polyethylene terephthalate (PET) bottle by the following process:

1. Preparation of raw materials containing BHET

A used PET bottle containing 10 wt% of a colored PET bottle was charged into a wet pulverizer provided with cutter knives and pulverized to obtain flakes having an average size of 8 mm square. The flakes were taken out. Then, 200 kg of a 4 wt% NaOH aqueous solution was added to 50 kg of the flakes, heated to 80 to 85 °C and stirred to wash the flakes for 30 minutes. Thereafter, the flakes were separated into solid and liquid and washed with water to remove the NaOH content. The flakes were then rinsed with pure water, centrifuged and dehydrated, and then vacuum dried.
30 kg of the dried flakes were supplied, together with 168 kg of ethylene glycol (hereinafter referred to as "EG") and 108 g of $Mg (OH)_2$ as a catalyst, to an autoclave equipped with a stirrer and a rectification column to obtain a mixed solution. Thereafter, nitrogen substitution and reduced pressure degassing were repeated three times, and depolymerization reaction was carried out for 1.5 hours at 195 to 200 °C under normal pressure while a fraction having a boiling point lower than that of EG was removed outside the system from the top of the rectification column. After the depolymerization reaction was completed, the temperature of the mixed solution was cooled to 97-98 °C in 45 minutes with stirring. After cooling, hot coarse particles were filtered with a stainless steel wire mesh filter having an average pore diameter of 60 μm and further filtered with a 1 μm fiber felt bag filter to remove suspended matters and precipitates in the mixed solution.
The above mixed solution was supplied to a jacket type vertical thin film evaporator equipped with a stirrer (Model "RF-6" of UIC GmbH) and the low boiling point component was evaporated from the solution under conditions of a jacket temperature of 150°C and an internal pressure of the evaporator main body of 533 Pa to obtain a first stage concentrate.
The first stage concentrated solution was again supplied to the above jacket type vertical thin film evaporator and the remaining low boiling point component in the first stage concentrate was evaporated under the condition that the jacket temperature was 150°C and the internal pressure of the evaporator main body was 133 Pa to obtain a second stage concentrate (high boiling point component).
The second stage concentrate (high-boiling point component) was supplied to a jacket type vertical thin film evaporator equipped with a stirrer (Model "RF-6" of UIC GmbH) and the weight ratio of the BHET evaporated fraction to evaporation residue (still residue) was set to be 8: 2 under the setting conditions that a jacket temperature was 202°C and an internal pressure of the evaporator main body was 13 Pa.
Next, 27 kg of the obtained crude BHET was dissolved in 108 kg of hot water at 70°C. This aqueous solution was supplied to a crystallization tank, rapidly cooled from 70 °C to 65°C, slowly cooled from 65 °C to 40°C for 5 hours and gradually cooled from 40 °C to 20°C for 2 hours to thereby crystallize and precipitate BHET crystals. Thereafter, a solid-liquid separation of the aqueous solution was performed using a vertical type centrifugal separator while maintaining the aqueous solution at 20°C. The purified BHET remained in the centrifugal separator was washed with pure water of 15°C and then subjected to solid-liquid separation again.
The purified BHET separated as described above was analyzed by a gas chromatography with the result that the purity was 96.3 wt%, the acid value of purified BHET was 2.3 mg KOH/g and the melting point was 111°C. Columns and measurement temperature conditions used for the gas chromatography analysis in this step and in each of the following steps are as follows:

- Column: DB-1 (made by Agilent Technologies, 30 m × 0.25 mm, film thickness 0.25 μm)
- Temperature conditions: raised from 150°C to 300 °C at 5 °C/min and held at 300°C for 15 minutes
- Carrier gas: helium

2. Nuclear hydrogenation of BHET

80 g of the purified BHET (raw material containing BHET) obtained above, 2.4 g of a carbon carrier catalyst (5% Ru/C) supporting 5 wt% ruthenium and 120 g of EG as a solvent (liquid medium) were charged into a 0.5 L electromagnetic stirring type autoclave. The weight ratio of the ruthenium-supported carbon carrier to BHET was 3.1 wt%, with the weight ratio of EG to BHET being 155.8 wt%.
After evacuating the inside of the autoclave under reduced pressure, a nuclear hydrogenation of BHET was carried out for 2.1 hours while hydrogen gas was continuously supplied into the autoclave so that the partial pressure of hydrogen gas in the autoclave could be maintained at 3 MPa with the temperature in the autoclave being at 130 °C.
Thereafter, the interior of the autoclave was cooled to room temperature, and the ruthenium-supported carbon

carrier catalyst was filtered out (separated) from the reaction product to obtain a reaction mixture solution.

Further, EG was distilled off from the reaction mixture solution by evaporation under reduced pressure to obtain a crude 1,4-cyclohexanedicarboxylic acid bis (2-hydroxyethyl) ester (crude BHEC). Such a crude BHEC was used as a raw material containing cis-BHEC in each of the following Examples and Comparative Examples shown below.

The obtained crude BHEC (raw material) contained 1,4-cyclohexanedicarboxylic acid bis (2-hydroxyethyl) ester (BHEC) having a purity of 89.3 wt% and a trans isomer ratio of 19.6%.

In addition, based on the contents (wt%) of cis-BHEC and trans-BHEC obtained by gas chromatography analysis of raw materials and products (crude trans-BHEC described later), the ratios of trans-isomer and cis-isomer were calculated from the following equation.

$$\text{Trans isomer ratio (\%)} = \text{Content of trans-BHEC} / (\text{Cis-BHEC content} + \text{trans-BHEC content}) \times 100$$

$$\text{Cis isomer ratio (\%)} = 100 - \text{trans isomer ratio}$$

Further, the reaction rate (%) of cis-BHEC was calculated from the following formula based on the cis-isomer ratio (%) of the raw material and the product obtained by the above formula.

$$\text{Reaction rate (\%) of cis-BHEC} = (\text{Cis isomer ratio of raw material} - \text{ratio of cis isomer of product}) / \text{ratio of cis isomer of raw material} \times 100$$

3. Cis-BHEC Isomerization

40 g of the crude BHEC (raw material containing cis-BHEC) obtained as described above and 1g of barium oxide powder (Commercially available special grade) as a catalyst and 60 g of ethylene glycol (EG) as a solvent were charged into a 0.3 L electromagnetic stirring type stainless steel autoclave equipped with a thermometer and a pressure gauge. The weight ratio of barium oxide powder to cis-BHEC was about 3.5 wt%, and the weight ratio of EG to cis-BHEC was about 209 wt%.

After evacuating the inside of the autoclave under reduced pressure, the interior of the autoclave was replaced with nitrogen gas. Thereafter, the mixture in the autoclave was stirred at 260 °C for 7 hours to perform isomerization of cis-BHEC.

Thereafter, the interior of the autoclave was cooled to room temperature, and the barium oxide powder was separated by filtration (separation) from the reaction system to obtain a reaction mixture solution.

Further, EG was distilled off from the reaction mixture by evaporation under reduced pressure to obtain 37.5 g of crude trans-1,4-cyclohexanedicarboxylic acid bis (2-hydroxyethyl) ester (crude trans-BHEC). As a result of gas chromatography analysis, the BHEC purity of the crude trans-BHEC was 83.9 wt%, the trans-isomer ratio was 67.7%, and the reaction rate of cis-BHEC was 59.8%.

(Example 2)

Isomerization was carried out in the same manner as in Example 1 except that 1 g of calcium oxide powder (commercially available special grade) was used as a catalyst instead of barium oxide powder to obtain a reaction mixture. Thereafter, the same post-treatment as in Example 1 was carried out to obtain a crude transformer-BHEC. The crude trans-BHEC obtained in this example was 38.2 g. As a result of gas chromatography analysis, the BHEC purity of the crude transformer-BHEC was 77.7 wt%, the trans isomer ratio was 68.8%, and the reaction rate of cis-BHEC was 61.2%.

(Example 3)

Isomerization was carried out to obtain a reaction mixture solution in the same manner as in Example 1 except that the amount of barium oxide powder was 2 g (weight ratio to cis-BHEC: about 7 wt%), the reaction temperature in

the autoclave was 200 °C and the stirring time (reaction time) was 10 hours. Thereafter, the same post-treatment as in Example 1 was performed to obtain a crude transformer-BHEC. The crude trans-BHEC obtained in this example was 38.8 g. As a result of gas chromatography analysis, the BHEC purity of the crude trans-BHEC was 85.8 wt%, the trans isomer ratio was 66.9%, and the reaction rate of cis-BHEC was 58.8%.

(Example 4)

The isomerization was carried out to obtain a reactant mixed solution in the same manner as in Example 3 except that the amount of EG as a solvent was 30 g (weight ratio to cis-BHEC: about 105 wt%) and the reaction temperature in the autoclave was 180 ° C. The hue of this reaction mixture was the lowest in a degree of coloring compared to the reaction mixture obtained in Example 1 (reaction temperature 260 °C) and Example 3 (reaction temperature 200 °C). Thereafter, the same post-treatment as in Example 1 was carried out to obtain a crude transformer-BHEC. The crude trans-BHEC obtained in this example was 39.0 g. As a result of gas chromatography analysis, the BHEC purity of the crude trans-BHEC was 88.8 wt%, the trans isomer ratio was 66.5 %, and the reaction rate of cis-BHEC was 58.3 %.

(Comparative Example 1)

Isomerization was carried out in the same manner as in Example 1 except that 1 g of aluminum oxide powder (commercially available special grade) was used as a catalyst instead of barium oxide powder to obtain a reaction mixture solution. Thereafter, the same post-treatment as in Example 1 was carried out to obtain a crude transformer-BHEC. The crude trans-BHEC obtained in this comparative example was 33.5 g. As a result of gas chromatography analysis, the BHEC purity of the crude trans-BHEC was 83.0 wt%, the trans isomer ratio was 51.7 %, and the reaction rate of cis-BHEC was 39.9 %.

(Comparative Example 2)

Isomerization was carried out in the same manner as in Example 1 except that 1 g of zinc oxide powder (commercially available special grade) was used instead of the barium oxide powder as a catalyst to obtain a reaction mixture solution. Thereafter, the same post-treatment as in Example 1 was carried out to obtain a crude transformer-BHEC. The crude trans-BHEC obtained in this comparative example was 34.2 g. As a result of gas chromatography analysis, the BHEC purity of the crude trans-BHEC was 82.0 wt%, the trans isomer ratio was 35.7 %, and the reaction rate of cis-BHEC was 20.0 %.

The composition of the crude trans-BHEC obtained in each example and each comparative example was analyzed using gas chromatography, and the results are shown in Table 1.

In addition, the color of the reaction mixture liquid of each example and each comparative example was visually confirmed and its coloring level was evaluated according to the following criteria. The results are shown in Table 1.

A: light yellow
B: Yellow
C: Brown

[Table 1]

| | | Example1 | Example2 | Example3 | Example4 | Comparative Example1 | Comparative Example2 |
|---|---|---|---|---|---|---|---|
| Raw material | Amount of use (g) | 40 | | | | | |
| | BHEC purity (wt%) | 89.3 | | | | | |
| | Cis isomer ratio (%) | 80.4 | | | | | |
| | Trans isomer ratio (%) | 19.6 | | | | | |
| | BHEC content (g) | 35.7 | | | | | |
| Catalyst type | | BaO powder | CaO powder | BaO powder | BaO powder | Al2O3 powder | ZnO powder |
| Amount of catalyst use (wt% to cis-BHEC) | | 3.5 | 3.5 | 7.0 | 7.0 | 3.5 | 3.5 |
| Amount of solvent (EG) use (wt% to cis-BHEC) | | 209 | 209 | 209 | 105 | 209 | 209 |
| Reaction temperature (°C) | | 260 | 260 | 200 | 180 | 260 | 260 |
| Reaction time (h) | | 7 | 7 | 10 | 10 | 7 | 7 |
| Color of reaction mixture solution | | C | C | B | A | C | C |
| GAS CHROMATOGRAPHY ANALYSIS RESULTS OF CRUDE TRANS-BHEC | | | | | | | |
| BHEC purity (wt%) | | 83.9 | 77.7 | 85.8 | 88.8 | 83.0 | 82.0 |
| Cis isomer ratio (%) | | 32.3 | 31.2 | 33.1 | 33.5 | 48.3 | 64.3 |
| Trans isomer ratio (%) | | 67.7 | 68.8 | 66.9 | 66.5 | 51.7 | 35.7 |
| Cis-BHEC reaction rate | | 59.8 | 61.2 | 58.8 | 58.3 | 39.9 | 20.0 |

Gas chromatograms of the reaction mixtures obtained in Example 1, Example 3 and Example 4 are shown in FIG. 1, FIG. 2 and FIG. 3, respectively.

As shown in Table 1, the ratio of the trans isomer in the crude trans-BHEC obtained in Examples 1 to 4 was 60 % or more, so that the trans-BHEC with high purity can be obtained from the raw material containing cis-BHEC. As such, since the high purity of trans-BHEC in the crude trans-BHEC was obtained with a small physical loss in the post-treatment process in the production processes of Examples 1 to 4, the yield of trans-BHEC was sufficiently high.

In addition, as shown in Table 1, the degree of coloring was lower in Example 3 than in Example 1, and the same in Example 4 was further lower than in Example 3. Moreover, it was found from the gas chromatogram of the reaction mixture obtained in each of Examples 1, 3 and 4 as shown in FIGS. 1 to 3, respectively, that the amount of ester hydrolysate and other by-products in the reaction mixture was small in the order of Examples 1, 3 and 4. Therefore, it was shown that if the reaction was carried out at the reaction temperature within the preferable range, the resultant reaction mixture had less coloration and less side reaction even at a lower reaction temperature as much as possible. Further, as long as the reaction temperature is a relatively low temperature of about 160 to 200 °C, the reaction can be carried out under normal pressure. Therefore, it is unnecessary to use a high pressure apparatus, and a relatively inexpensive reaction apparatus can be used, which is industrially advantageous.

On the other hand, in Comparative Examples 1 and 2 in which an amphoteric oxide (aluminum oxide, zinc oxide) was used as a catalyst, cis-BHEC in the raw material was not sufficiently isomerized, and the purity of the obtained trans-BHEC was low, and the yield could not be increased sufficiently.

Incidentally, isomerization was carried out in the same manner as in Example 1 using raw materials containing cis-BHAC of cis-1,4-cyclohexanedicarboxylic acid bis (2-hydroxypropyl) ester (cis-BHPC) and cis-1,4-cyclohexanedicarboxylic acid bis (2-hydroxybutyl) ester (cis-BHBC), instead of the raw material containing cis-BHEC, to obtain crude trans-BHAC. As a result, trans-BHAC with high purity could be obtained from any of the cis-BHAC containing raw materials.

## Claims

1. A process for producing trans-cyclohexanedicarboxylic acid bis (2-hydroxyalkyl) ester comprising a step of isomerizing cis-cyclohexanedicarboxylic acid bis (2-hydroxyalkyl) ester by contacting with a basic oxide to obtain trans-cyclohexanedicarboxylic acid bis (2-hydroxyalkyl) ester.

2. The process according to claim 1, wherein the isomerization is carried out at temperatures of 160 to 300 °C.

3. The process according to claim 1 or 2, wherein the weight ratio of the cis-cyclohexanedicarboxylic acid bis (2-hydroxyalkyl) ester to the basic oxide is 100: 0.1 to 100: 10.

4. The process according to any one of claims 1 to 3, wherein the isomerization is carried out in a state in which the cis-cyclohexanedicarboxylic acid bis (2-hydroxyalkyl) ester and the basic oxide are mixed in a liquid medium.

5. The process according to claim 4, wherein the liquid medium comprises at least one of a diol and an aprotic polar solvent.

6. The process according to claim 5, wherein the liquid medium comprises at least one of ethylene glycol, propylene glycol, butanediol, N, N-dimethylformamide, dimethylsulfoxide and 1,3-dimethyl-2-imidazolidinone.

7. The process according to any one of claims 1 to 6, wherein the basic oxide comprises at least one of alkali metal oxides and alkaline earth metal oxides.

8. The process according to claim 7, wherein the basic oxide comprises at least one of barium oxide, calcium oxide and magnesium oxide.

9. The process according to any one of claims 1 to 8, wherein the cis-cyclohexanedicarboxylic acid bis (2-hydroxyalkyl) ester is cis-1,4-cyclohexanedicarboxylic acid bis (2-hydroxyethyl) ester.

10. The process according to claim 9, wherein the cis-1,4-cyclohexanedicarboxylic acid bis (2-hydroxyethyl) ester is produced by depolymerizing a raw material containing polyethylene terephthalate to obtain terephthalic acid bis (2-hydroxyethyl) ester, and then nuclear hydrogenating the terephthalic acid bis (2-hydroxyethyl) ester.

11. The process according to claim 10, wherein the nuclear hydrogenation is carried out by reacting the terephthalic

acid bis (2-hydroxyethyl) ester with hydrogen gas in the presence of a ruthenium catalyst.

**12.** The process according to claim 11, wherein the nuclear hydrogenation is carried out in a state where the terephthalic acid bis (2-hydroxyethyl) ester and the ruthenium catalyst are mixed in a liquid medium.

**Patentansprüche**

**1.** Verfahren zur Herstellung von trans-Cyclohexandicarbonsäure-bis(2-hydroxyalkyl)ester, umfassend einen Schritt der Isomerisierung von cis-Cyclohexandicarbonsäure-bis(2-hydroxyalkyl)ester durch Kontaktieren mit einem basischen Oxid, um so trans-Cyclohexandicarbonsäure-bis(2-hydroxyalkyl)ester zu erhalten.

**2.** Verfahren gemäß Anspruch 1, bei dem die Isomerisierung bei Temperaturen von 160 bis 300°C durchgeführt wird.

**3.** Verfahren gemäß Anspruch 1 oder 2, bei dem das Gewichtsverhältnis des cis-Cyclohexandicarbonsäure-bis(2-hydroxyalkyl)esters zum basischen Oxid 100:0,1 bis 100:10 beträgt.

**4.** Verfahren gemäß mindestens einem der Ansprüche 1 bis 3, bei dem die Isomerisierung in einem Zustand durchgeführt wird, in dem der cis-Cyclohexandicarbonsäure-bis(2-hydroxyalkyl)ester und das basische Oxid in einem flüssigen Medium gemischt sind.

**5.** Verfahren gemäß Anspruch 4, bei dem das flüssige Medium mindestens eines aus einem Diol und einem aprotischen polaren Lösungsmittel umfasst.

**6.** Verfahren gemäß Anspruch 5, bei dem das flüssige Medium mindestens eines aus Ethylenglykol, Propylenglykol, Butandiol, N,N-Dimethylformamid, Dimethylsulfoxid und 1,3-Dimethyl-2-imidazolidinon umfasst.

**7.** Verfahren gemäß mindestens einem der Ansprüche 1 bis 6, bei dem das basische Oxid mindestens eines aus Alkalimetalloxiden und Erdalkalimetalloxiden umfasst.

**8.** Verfahren gemäß Anspruch 7, bei dem das basische Oxid mindestens eines aus Bariumoxid, Calciumoxid und Magnesiumoxid umfasst.

**9.** Verfahren gemäß mindestens einem der Ansprüche 1 bis 8, bei dem der cis-Cyclohexandicarbonsäure-bis(2-hydroxyalkyl)ester cis-1,4-Cyclohexandicarbonsäure-bis(2-hydroxyethyl)ester ist.

**10.** Verfahren gemäß Anspruch 9, bei dem der cis-1,4-Cyclohexandicarbonsäure-bis(2-hydroxyethyl)ester durch Depolymerisation eines Polyethylenterephthalat-haltigen Rohmaterials, um Terephthalsäure-bis(2-hydroxyethyl)ester zu erhalten, und anschließende Kernhydrierung des Terephthalsäure-bis(2-hydroxyethyl)esters hergestellt wird.

**11.** Verfahren gemäß Anspruch 10, bei dem die Kernhydrierung durch Umsetzung des Terephthalsäure-bis(2-hydroxyethyl)esters mit Wasserstoffgas in Gegenwart eines Rutheniumkatalysators durchgeführt wird.

**12.** Verfahren gemäß Anspruch 11, bei dem die Kernhydrierung in einem Zustand durchgeführt wird, in dem der Terephthalsäure-bis(2-hydroxyethyl)ester und der Rutheniumkatalysator in einem flüssigen Medium gemischt sind.

**Revendications**

**1.** Procédé pour la production d’ester bis(2-hydroxyalkyle) d’acide trans-cyclohexanedicarboxylique comprenant une étape d’isomérisation de l’ester bis(2-hydroxyalkyle) d’acide cis-cyclohexanedicarboxylique par mise en contact avec un oxyde basique pour obtenir l’ester bis(2-hydroxyalkyle) d’acide trans-cyclohexanedicarboxylique.

**2.** Procédé selon la revendication 1, dans lequel l’isomérisation est effectuée à des températures allant de 160 à 300 °C.

**3.** Procédé selon la revendication 1 ou la revendication 2, dans lequel le rapport pondéral de l’ester bis(2-hydroxyalkyle) de l’acide cis-cyclohexanedicarboxylique à l’oxyde basique est de 100:0,1 à 100:10.

4. Procédé selon l'une quelconque des revendications 1 à 3, dans lequel l'isomérisation est effectuée dans un état dans lequel l'ester bis(2-hydroxyalkyle) de l'acide cis-cyclohexanedicarboxylique et l'oxyde basique sont mélangés dans un milieu liquide.

5. Procédé selon la revendication 4, dans lequel le milieu liquide comprend au moins un parmi un diol et un solvant polaire aprotique.

6. Procédé selon la revendication 5, dans lequel le milieu liquide comprend au moins un parmi l'éthylène glycol, le propylène glycol, le butanediol, le N,N-diméthylformamide, le diméthylsulfoxyde et la 1,3-diméthyl-2-imidazolidinone.

7. Procédé selon l'une quelconque des revendications 1 à 6, dans lequel l'oxyde basique comprend au moins un parmi des oxydes de métaux alcalins et des oxydes de métaux alcalino-terreux.

8. Procédé selon la revendication 7, dans lequel l'oxyde basique comprend au moins un parmi l'oxyde de baryum, l'oxyde de calcium et l'oxyde de magnésium.

9. Procédé selon l'une quelconque des revendications 1 à 8, dans lequel l'ester bis(2-hydroxyalkyle) d'acide cis-cyclohexanedicarboxylique est l'ester bis(2-hydroxyéthyle) d'acide cis-1,4-cyclohexanedicarboxylique.

10. Procédé selon la revendication 9, dans lequel l'ester bis(2-hydroxyéthyle) de l'acide cis-1,4-cyclohexanedicarboxylique est produit par la dépolymérisation d'une matière première contenant du polyéthylène téréphtalate pour obtenir l'ester bis(2-hydroxyéthyle) de l'acide téréphtalique, puis par l'hydrogénation nucléaire de l'ester bis(2-hydroxyéthyle) de l'acide téréphtalique.

11. Procédé selon la revendication 10, dans lequel l'hydrogénation nucléaire est effectuée par la mise en réaction de l'ester bis(2-hydroxyéthylique) de l'acide téréphtalique avec de l'hydrogène gazeux en présence d'un catalyseur au ruthénium.

12. Procédé selon la revendication 11, dans lequel l'hydrogénation nucléaire est effectuée dans un état dans lequel l'ester bis(2-hydroxyéthylique) de l'acide téréphtalique et le catalyseur au ruthénium sont mélangés dans un milieu liquide.

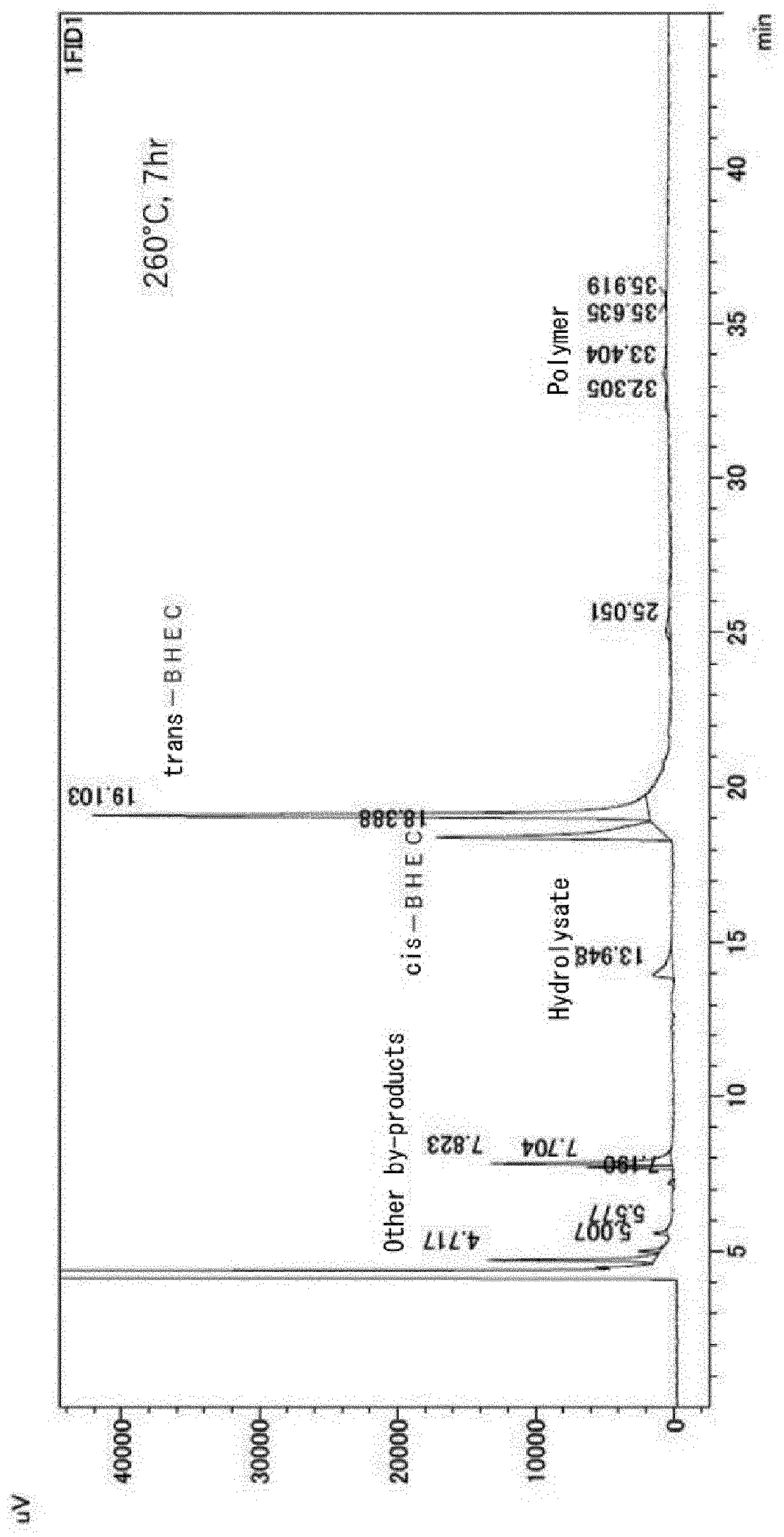
260°C, 7hr
1FID1
uV
min
trans−BHEC
cis−BHEC
Hydrolysate
Other by-products
Polymer
19.103
18.388
13.948
25.051
32.305
33.404
35.635
35.919
4.717
5.007
5.577
7.190
7.704
7.823
0
10000
20000
30000
40000
5
10
15
20
25
30
35
40

FIG. 1

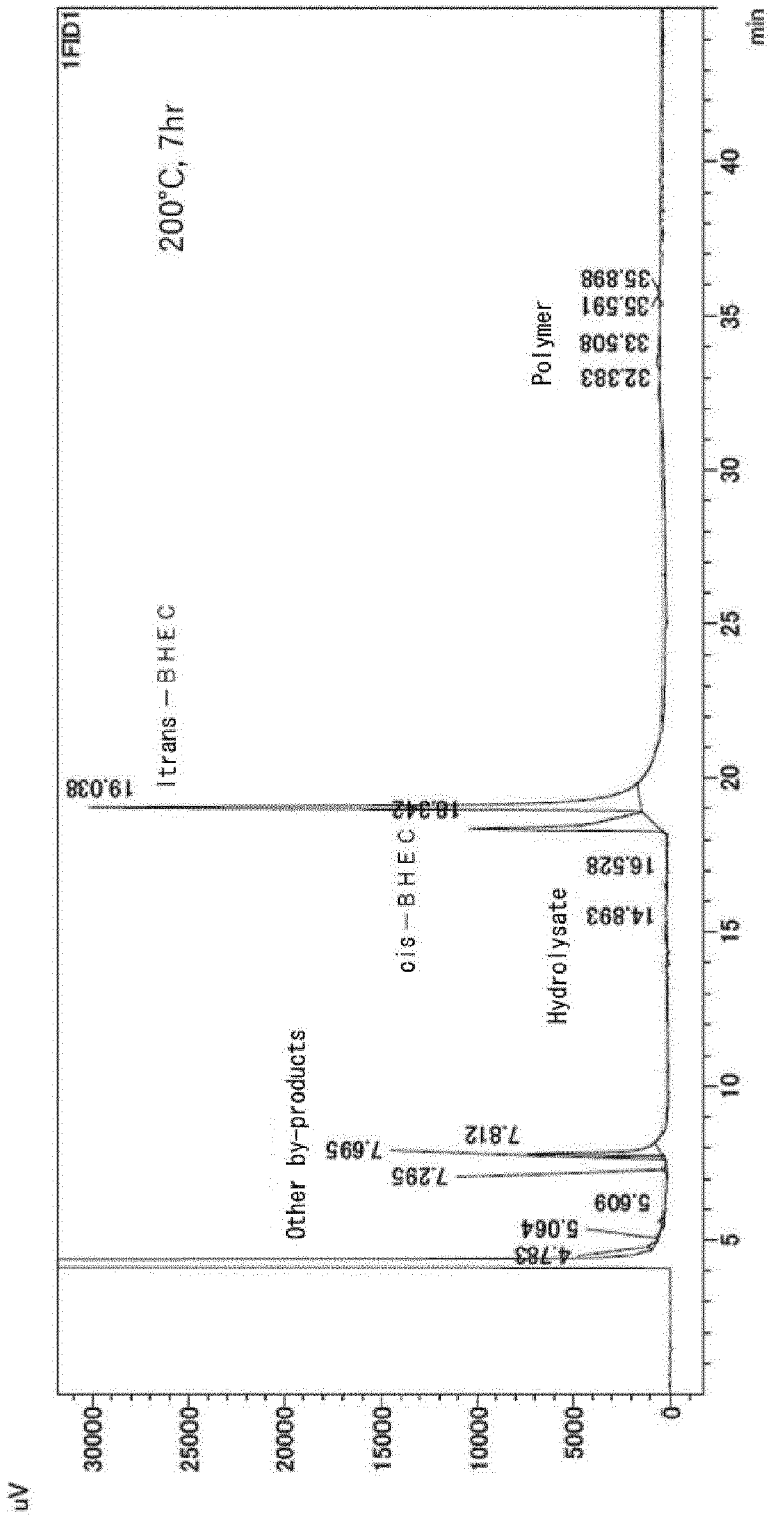

200°C, 7hr
1FID1
uV
min
trans－BHEC
cis－BHEC
Hydrolysate
Polymer
Other by-products
19.038
18.342
16.528
14.893
7.812
7.695
7.295
5.609
5.064
4.783
32.383
33.508
35.591
35.898


FIG. 2

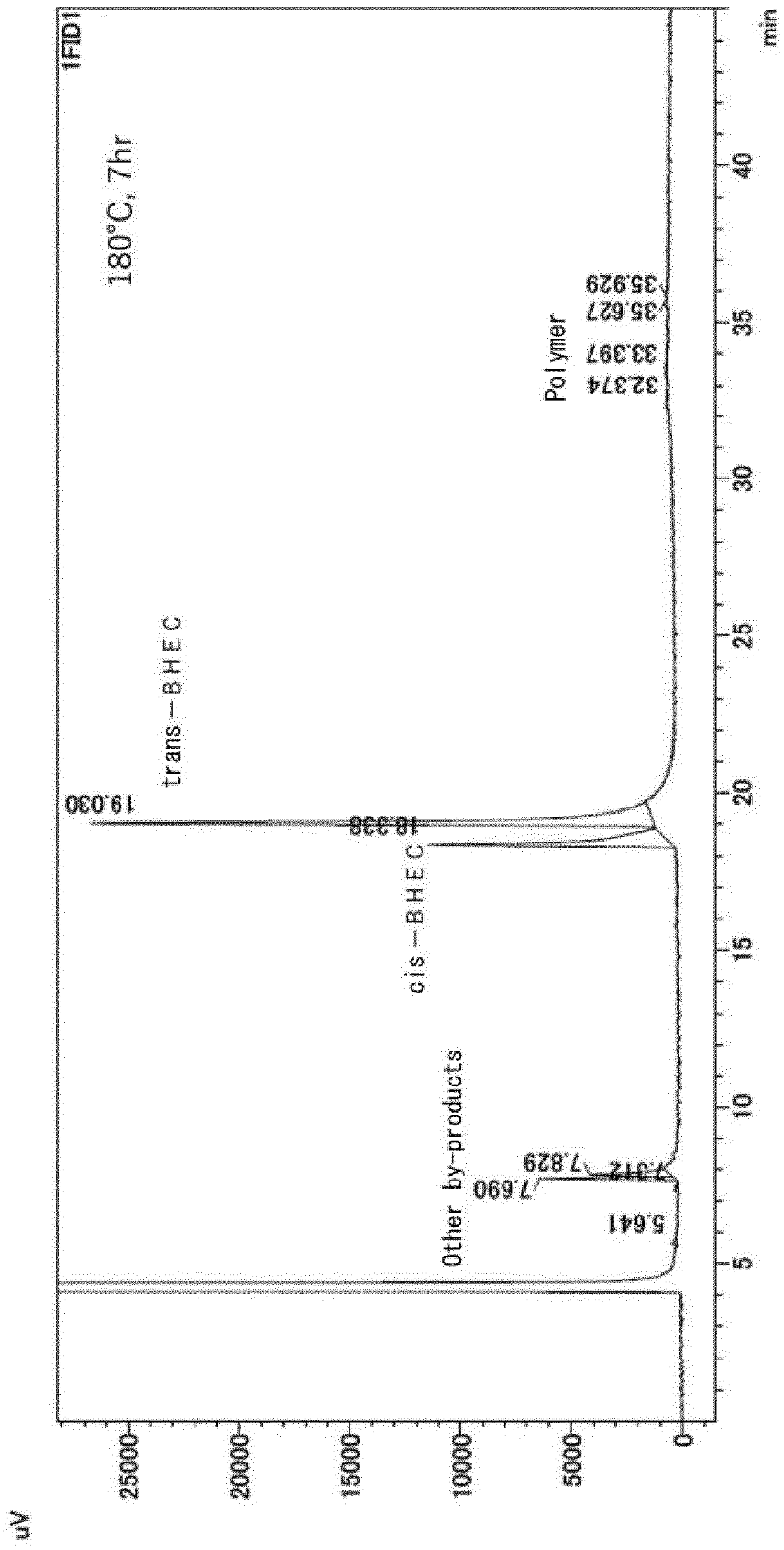
1FID1
180°C, 7hr
trans-BHEC
19.030
18.338
cis-BHEC
Other by-products
7.690
7.829
7.312
5.641
Polymer
32.374
33.397
35.627
35.929
min
uV
25000
20000
15000
10000
5000
0
5
10
15
20
25
30
35
40

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104003840 A
- US 5817731 A
- JP 2000053802 A
- JP 2008088096 A